# EUROPEAN PATENT SPECIFICATION

(11) **EP 0 600 101 B1**
(45) Date of publication and mention of the grant of the patent: **04.11.1998**
(21) Application number: 93913663.6
(22) Date of filing: 12.05.1993
(51) Int. Cl.: B03C 3/14, A61L 9/22, B03C 3/01, B03C 3/38

(54) **DEVICE FOR BIOLOGICAL CLEANING AND FILTRATION OF AIR**
VORRICHTUNG ZUR BIOLOGISCHE REINIGUNG UND FILTRATION VON LUFT
DISPOSITIF DE NETTOYAGE ET DE FILTRAGE BIOLOGIQUE D'AIR

(30) Priority: 13.05.1992 RU 5048011
(43) Date of publication of application: 08.06.1994
(73) Proprietor: VOLODINA, Elena Vladimirovna, Mosco, 123252 (RU); NAGOLKIN, Alexandr Vladimirovich, Moscow, 117330 (RU)
(72) Inventor: VOLODINA, Elena Vladimirovna, Mosco, 123252 (RU); NAGOLKIN, Alexandr Vladimirovich, Moscow, 117330 (RU)
(74) Representative: Godwin, Edgar James
(86) International application number: PCT/RU93/00107
(87) International publication number: WO 93/23171

(56) References cited:
- AU-A- 498 233
- DE-A- 2 658 510
- FR-A- 1 410 881
- FR-A- 2 220 311
- US-A- 3 735 560
- US-A- 3 798 879
- US-A- 4 133 653
- US-A- 4 376 642

## Description

### Field of the Invention

The present invention relates to air cleaning plants and more particularly to apparatus for biological cleaning and filtration of air.

### State of the Prior Art

An apparatus for biological cleaning of air is known from US Patent A 3,918,939, said apparatus comprising a housing, at least one nondischarging precipitation electrode and at least one discharge electrode.

In the above apparatus, the particles suspended in air are forced to enter the working zone where they are exposed to electric forces influencing on the magnitude and polarity sign of their charge. Depending on the magnitude of this charge and its sign, the particles are precipitated on the electrodes and collecting plates so that cleaning of air is ensured thereby.

In that apparatus the efficiency of filtration depends on the strength of the electric field, the air flow rate and properties of the particles, and the efficiency is proportional to the consumption of energy and, as a rule, is low.

A similar apparatus for cleaning and filtration of air is known from US patent A 1,346,207, said apparatus comprising cartridges with filter elements of basaltic cardboard with water-repellent impregnation that are arranged across the flow in parallel to each other.

In the above apparatus, air containing the particles to be removed is forced through plates. Therewith, those particles are retained mechanically which have their geometrical dimensions to exceed the dimensions or cardboard pores.

In addition to this, the water-repellent impregnation of cardboard gives rise to the formation of electrostatic charge of the particles to cause their precipitation on the walls of the pores. However, the cardboard used has low permeability and small size of pores, As a result, this apparatus has a short service life since the pores get rapidly clogged with the particles thus retained, and the water-repellent impregnation looses its tribostatic properties with time. Besides, a substantial positive pressure is required to be built up in the system to ensure the operation of such an apparatus.

The ionizator (ionizer) includes oppositely charged electrodes.

The fine filter comprises successive layers of woven filtering material and a reservoir filled with liquid.

Air to be cleaned is fed forcibly to the apparatus and passes through the coarse filter where the particles are retained which have the size thereof to exceed the size of the cells in the woven material. The particles remaining in the air then enter the ionization zone of the ionizator together with the air flow, where they acquire an electric charge and deposit on the woven fine filter and on the surface of liquid.

In that apparatus, a combined mechanism of collecting the particles is realised: mechanical capture and electric forces, as well as molecular forces.

Since the efficiency of cleaning is determined by the quantity of particles thus removed, it is rather important to do so that each of the components of the cleaning apparatus would retain as many particles as possible. However, in this design the quantity of particles retained on the plate of the coarse filter will, like also in the apparatus described herein above, depend on the size of the cells in filtering material. i.e., the smaller the size of the cells, the higher will be the efficiency of filtration (smaller particles removed), but simultaneously there is an increase in aerodynamic drag, and this leads to a shorter service life since the filtering material becomes dirty more rapidly and higher air pressure is required to overcome the drag of the filter- a factor which involves additional consumption of energy.

FR-A-2 220 311 discloses air cleaning apparatus in which an electrostatic precipitator is positioned downstream of an ionizer. FR-A-1 410 881 discloses air cleaning apparatus according to the preamble of claim 1.

All the designs described herein above show low efficiency of biological cleaning of air and shorter service life; besides, their characteristics are unstable and depend on "fouling" of filtering materials. It should be also pointed out that the ionizators of electrostatic precipitators release large quantities of ozone and nitrogen oxides, and this makes it difficult to use them in human environment.

### Summary of the Invention

The object of this invention is to provide an apparatus for cleaning and filtration of air, allowing to retain most of the particles to be removed without an increase in aerodynamic drag of the filtering plates, so that as many microorganisms and virus are inactivated therewith as possible and a low quantity of ozone leaves the apparatus.

The invention provides air cleaning apparatus as set forth in claim 1.

The apparatus comprises a coarse filter, an ionizator (ionizer) formed by at least a pair of electrodes connected to the terminals of opposite polarity of a power source and a fine filter, which are installed in this succession along the path of the gas (air) flow. Use is made of a coarse electrostatic precipitator consisting of at least three plates arranged across the gas flow adjacently to each other, the outermost ones of which are made of gas-permeable current-conducting material and connected to the terminals of opposite polarity of the power source, whereas an inner plate located therebetween is made of high-porous electret material. Therewith, as such gas-permeable current-conducting material it is reasonable to use cellular metal having open porosity of at least 85% and pore diameter of O.3 : 5 mm, and as such high porous electret material it is reasonable to use polyurethane foam having open porosity of at least 85% and pore diameter of 0.3 to 5 mm.

It is desirable that the size of pores in the filter elements be selected O.3 to 5 mm since a reduction of the size below O.3 mm leads to a considerable rise in aerodynamic drag, whereas an increase thereof above 5 mm reduces the influence of electric forces on the particles, and this in turn impairs efficiency of their removal.

In case when the ionizator comprises a discharge electrode shaped as a needle arranged along the gas flow, and a nondischarging electrode shaped essentially as a hollow cylinder arranged coaxial relative to the needle, it is desirable that the cylindrical electrode has the end face thereof to abut on the nearest current-conducting plate so that the plate overlaps the end face completely.

The fine filter is reasonable to be made similarly to the coarse filter. Therewith, the plates of the fine filter can be made of metal and arranged to be disposed either across the gas flow or in parallel thereto. In this case, in order to ensure a minimum weight and improve the conditions for draining the charge off, it is preferable to make the current-conducting plates of the fine filter perforated.

Based on conditions of packaging, for instance, with the housing made as a cylinder, the plates of the fine filter can also be made as cylinders arranged to be disposed coaxially.

In the apparatus for cleaning and filtration of air it is reasonable to install an additional ionizator which has the design thereof similar to that of the main one. Therewith, the pairs of electrodes of the main and additional ionizators should be connected to the power source so that the nondischarging electrode of the first ionizator and the needle of the second ionizator are connected to the same terminal of the power source, and the needle of the first ionizator and the nondischarging electrode of the second ionizator to the terminal of the power source having the opposite sign.

### Brief Description of the Drawings

Hereinafter the invention will be described by way of examples in more detail with reference to the accompanying drawings illustrating specific embodiments thereof, in which according to the present invention:
Fig. 1 shows schematically a general view of an embodiment of apparatus for biological cleaning and filtration of air, in longitudinal section;
Fig. 2 shows an embodiment of this apparatus, with parallel plates in the fine filter, in longitudinal section;
Fig. 3 shows a fine filter with plates shaped as coaxial cylinders, end view, in cross-section;
Fig. 4 shows an embodiment with an additional ionizator, in longitudinal section;
Fig. 5 shows an embodiment in which the cylindrical electrodes of two ionizators are made to have different internal diameters, in longitudinal section;
Fig. 6 shows schematically an ionizator having a plurality of electrode pairs, in longitudinal section; and
Fig. 7 is a section on line VII-VII in Fig. 6.

### Best Mode for Carrying out the Invention

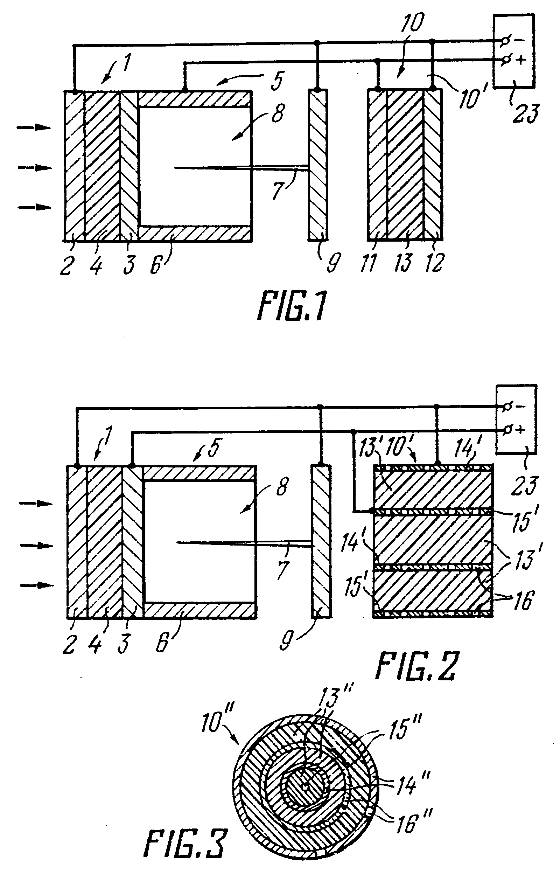

With reference to Fig. 1, the apparatus for biological cleaning and filtration of air, according to the invention, comprises a coarse filter 1 formed by two plates 2 and 3 made or gas-permeable current-conducting material, preferably cellular metal. This material is characterised by good electric conductivity, high porosity, stable structure. Inserted between these plates 2 and 3 is a plate 4 of dielectric, made of high-porous electret material such as, for instance, polyurethane foam. This material is characterised by high porosity (up to 98%),stable structure, good electric and tribostatic properties.

With this, it is desirable to have the size of pores selected on the basis of meeting the requirements of maximum permeability and sufficiency of electrostatic forces for precipitation of charged particles.

The current-conducting plates 2 and 3 are connected electrically to the terminals of opposite polarity (+, -) of a power source (23).

Located downstream of the last plate 3 in the path of gas flow is an ionizator (ionizer) 5 formed by a nondischarging electrode 6 and a discharge electrode 7. With this, the nondischarging electrode 6 is made as a cylinder 6 forming an ionization chamber 8, whereas the discharge electrode is made as a needle 7 arranged to be disposed along the axis of the above-mentioned cylinder 6. The end face of the cylindrical nondischarging electrode 6 abuts on the current-conducting plate 3 of the coarse filter 1 and is in an electrical contact with it.

Mounted at the base of the needle 7 is an additional plate 9 made of current-conducting high-porous material, like the plates 2 and 3. With this, the plate 9 is in electrical contact with the needle 7 and connected to the terminal of the power source, which has the sign opposite to that of the plate 3.

The additional plate 9 is designed for additional filtration and electrostatic precipitation of the charged particles, as well as for restricting the ionization zone and reducing the quantity of ozone released.

Located downstream of the additional plate 9 is a fine filter 10 which has its design similar to that of the coarse filter 1. The current-conducting plates 11 of the fine filter 10 are also made of cellular metal having porosity of at least 86%, whereas the dielectric plate arranged to be disposed between them is made of polyurethane foam. With this, the first current-conducting plate 11 in the path of gas flow, which is the nearest one to the additional plate 9, is connected to the terminal of the power source, which has the sign opposite to that of the plate 9, whereas the second current-conducting plate 12 is connected to the terminal of the power source, which has the same sign with that of the plate 9. Such connection of the current-conducting plates 11 and 12 of the fine filter 10 ensures higher probability of precipitation of the charged particles on the plates.

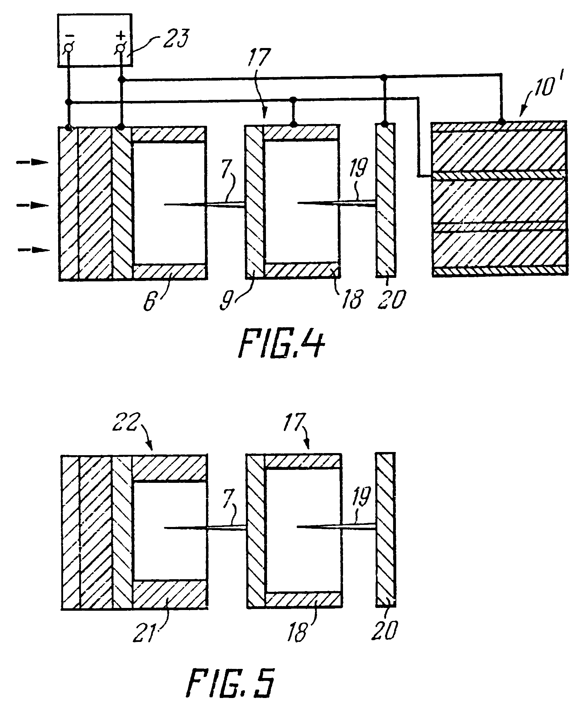

The embodiment of the apparatus shown in Fig. 2, in contrast to the apparatus shown in Fig. 1, comprises a fine filter 10' with plates 13', 14' and 15' arranged to be disposed along the path of gas flow. With this, the current-conducting plates 14' and 15' are made of sheet metal having perforations 16, whereas the dielectric plates 13' made of polyurethane foam. The current-conducting plates 14' and 15' are alternately connected to the unlike terminals of the power source. The perforations 16 of the plates 14' and 15' are desirable for better conditions of draining the charge off and for reducing the weight of the current-conducting plates.

The fine filter 10'' shown in Fig. 3 differs from the filter 10' shown in Fig. 2 by that plates 13'', 14'' and 15'' made as cylinders arranged to be disposed coaxial relative to each other, the cylinder coincident with the axis having therewith a zero diameter, i.e., it is essentially a stem. Such an embodiment increases the filtering surface without an increase in the overall dimensions of the filter, improves the capacity of the filter as well as ensures convenient packaging.

The embodiment of the apparatus shown in Fig. 4, in contrast to the embodiment shown in Fig. 1, comprises an additional ionizator 17 arranged to be disposed in parallel with the main ionizator. With this, the additional ionizator 17 is located so that its cylindrical electrode has the end face thereof to abut on the plate 9 and, respectively, it has the charge of the same sign as also the plate 9. With this, a needle 19 and a plate 20 are connected to the terminal of the power source having the opposite sign as compared with that of the charge of the cylindrical electrode 18. In this case, the air which flows through the apparatus and had to be cleaned, passes successively the ionization chambers having the corona discharges opposite to each other by their sign, and this causes recharging of the particles to be removed so that the probability of inactivating the microorganisms and viruses that are on them or suspended in the air is increased thereby and, hence, the efficiency of cleaning is thus improved.

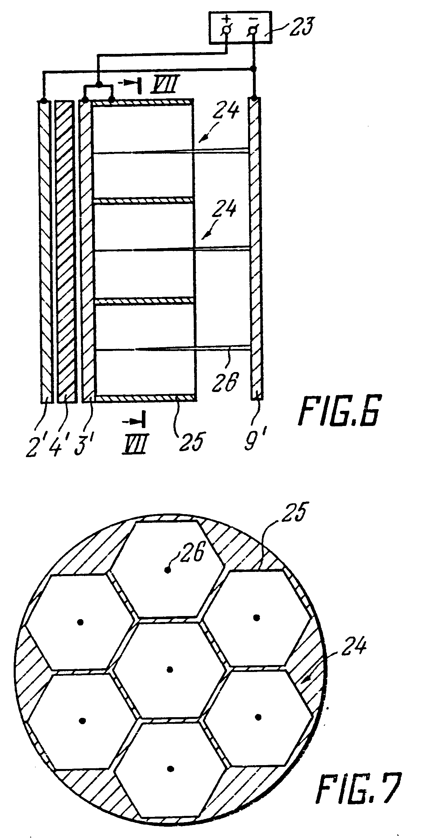

Fig. 5 in contrast to Fig. 4 shows schematically an embodiment of the cylindrical electrodes 18 and 21 which are inside the ionizators 17 and 22 arranged in series with one another and have different internal diameters. In this particular case, the internal diameter of the cylindrical electrode 21 of the ionizator 22 which is the first one in the path of the flow, is smaller than the internal diameter of the cylindrical electrode 18 of the second ionizator 17. Such an embodiment ensures optimization of the conditions for charging and recharging the particles, thus improving the efficiency of cleaning.

With this, the discharge electrodes of the main ionizator 17 and additional ionizator 22 are connected to the unlike terminals of the power source 23.

Figs. 6 and 7 show schematically an embodiment of an ionizator having a plurality of electrode pairs 24 forming thereby a plurality of ionization chambers. With this, the most compact structure is obtained when nondischarging electrodes 25 are shaped as hexahedral prisms, whereas the discharge electrodes are made as needles 26, and this also improves substantially the adaptability of the apparatus to streamlined manufacture. The current-conducting plate 3' of the coarse filter, which is the nearest one to the ionizators, is made to have such a size that it overlaps all the internal cavities of the prism-like electrodes 25 of the ionizator.

The apparatus illustrated in Fig. 1 operates in the following manner.

When the power source 23 is switched on, all the components connected to its terminals acquire an electric charge. These components include the plates 2 and 3 of the coarse filter and the plates 11 and 12 of the fine filter as well as the additional plates 9 and electrodes 6 of the ionizator. With this, every next component, or, if the components are connected with each other, the combination of components is connected to the unlike terminals of the power source so that an electric field, therefore, appears between plates 2 and 3 and plates 11 and 12 of the filters, which depolarizes the plates 4 and 13 made of dielectric, whereas a corona discharge appears between the electrodes 6 and 7 of the ionizator. The air to be cleaned passes through the current-conducting plates 2 and 3 and dielectric plates 4 of the coarse filter where the largest particles are retained due to mechanical capture and forces of electric attraction, and microorganisms and viruses are inactivated under the influence of the electric fields having the opposite sign.

With this, owing to the fact that porous cellular material or foam is used for the filtering plates in contrast to the known, more efficient retaining is ensured since it is determined not only by the size of the cell, in this case, by the size of the pore, but also by the thickness of the filter element. The configuration of a pore passage which is, as a rule, curvilinear, does not allow the particles to fly freely therethrough without coming into contact with its surface. And such a contact will lead either to retardation or to final retaining of the particles. Therewith not only those particles which have the size thereof to exceed that of the pores will be retained like it takes place in woven structures, but also the particles of smaller size because of their catching or hitting thereof into irregularities of the pore passage. The particles possessing an initial charge will stay for a longer time under the influence of the charged plates 2 and 3 of the coarse filter 1 due to the mechanism of retardation described above so that the probability, hence, increases that they will precipitate under the effect of electric forces.

The particles that have passed through the coarse filter 1 and, together with them, the microorganisms and viruses enter the ionizator 5. With the plate 3 of the coarse filter 1 arranged to be disposed immediately adjacent to the end face of the cylindrical electrode 6 of the ionizator 5, the most optimum distribution of the electric field is ensured inside the ionization chamber 8 so that the particles, microorganisms and viruses leaving the coarse filter find themselves straight away under its influence; the particles acquire an added charge, whereas the microorganisms, and viruses are subjected to inactivation. Depending on the charge acquired, the particles with inactivated microorganisms and viruses are partially precipitated on the surfaces of the electrodes 6 and 7.

As it is known, the corona discharge is accompanied by releasing ozone which in large quantities has an unfavourable effect on people. Since the plate 3 abuts tightly, on the ionization chamber 8, this creates, on the one hand, the better charging of particles and, on the other hand, the accompanied inside the chamber 8 ozone in contact with the plate decomposes.

Downstream the ionizator, the air flows through the additional plate 9 which is connected with the needle 7 of the ionizator and which, since it is made of cellular metal, also retains some of the particles, and inactivates microorganisms and viruses .In addition to this, the additional plate 9 restricts the ionization chamber 8 to some extent on the exit side, thus improving the charging conditions and reducing the quantity of ozone released therefrom. As a result, the total content of ozone in the air flow leaving the apparatus of the present invention does not exceed the values adopted for the ultimate allowable concentrations.

Further, the air with the particles, microorganisms and viruses still left suspended therein enter the fine filter 10 where, like in the coarse filter, mechanical and electrostatic precipitation of the particles, and inactivation of microorganisms and viruses also takes place. The air thus cleaned and made harmless by removal of microorganisms and viruses therefrom flows from the fine filter 10 to the outlet from the apparatus.

The apparatus shown in Figs. 2 and 3 differs from the apparatus shown in Fig. 1 only by an alternative embodiment of the fine filter 10 or 10''. The air is cleaned therein and the microorganisms and viruses inactivated just in the same manner like also in the apparatus shown in Fig. 1.

The operation or the apparatus shown in Fig. 4 goes on just in the same manner like also in the apparatus shown in Fig. 1, but the air to be cleaned passes additionally through another ionizator 17 installed in series with the main ionizator 5. The electrodes 17 and 18 of that ionizator 17 are connected to the power source 23 in such a manner that the corona discharge produced therein is opposite by its sign to the corona discharge in the main ionizator 5. Owing to this, the air thus being cleaned is additionally subjected to the influence of the charge opposite by its signs, and this is favourable for additional precipitation of the particles on the electrodes 18 and 19, whereas the recharging of microorganisms and viruses makes their inactivation more probable, i.e., they are killed in larger quantities under the influence of the field having the opposite sign.

The apparatus shown in Fig. 5 operates just in the same manner like the apparatus shown in Fig. 4 but, owing to the fact that the cylindrical electrodes 21 and 18 having different internal diameters are used in the main ionizator 22 and in the additional ionizator 17, respectively, the possibility is ensured for creating an electric field that is the most optimum one for retaining the particles depending on their size. This is attributed to the fact that in order to retain larger particles it is required to have a higher magnitude of electric charge which, in particular depends on the strength of the electric field determined in its turn by the distance between the discharge and nondischarging electrodes: the smaller this distance, the higher is the strength of the electric field.

For just the same reason, as well as to ensure safe operation from the viewpoint of the maximum strength of electric field possible in the ionization chambers, use is to be made of an jonizator packaged as shown in Fig. 6 to comprise a plurality of electrode pairs 24. In the apparatus shown in Fig. 6, the air to be cleaned flows into a plurality of ionization chambers formed by the electrode pairs 24 where the precipitation of particles and the inactivation of microorganisms and viruses take place. Further the air being cleaned passes through the components of the apparatus according to the present invention Just in the same manner like in the embodiment shown in FIG. 1.

Thus, an apparatus made in accordance with the present invention, thanks to the use of a coarse electrostatic precipitator made on the basis of cellular metal and polyurethane foam, as well as arranging it to be disposed adjacently to an ionizator have allowed to eliminate the shortcomings existing in the known cleaning apparatus: there is no substantial increase in the aerodynamic drag of the filtering plates, so that their service life does not become, therefore, shorter, there is an increase in the quantities of the particles retained, and the microorganisms and viruses inactivated, and the quantity of ozone released is reduced to the minimum.

In accordance with Fig. 1, a mock-up of the apparatus has been made, and its tests have been carried out with ambient air cleaned therein, as a result of which we have obtained the following data:
- efficiency of biological cleaning is 100%)
- filtration efficiency for the particles having the size within 0.01 to 10 microns is 96%; and
- power consumption is 45 W (with fan).

The apparatus is simple in operation, has, small dimensions and is superior to the known apparatus by its efficiency of the biological cleaning of the air.

### Industrial Usability

The apparatus can be used in medicine for biological cleaning of air in the blood transfusion laboratories, in the operating and re animating rooms, where a great number of patients are conveyed because it doesn't require any special time for the preliminary preparation of rooms, in Tuberculosis Hospitals, in Maternity Houses in the wards for the aids infected patients and the patients with weak immunity, in the Burn Centers, in stomatology; in pharmacology and microbiology; at locations where stringent requirements are imposed on purity and sterility of air, as well as in electronic industry and other industries where the quality of products manufactured is dependent upon the purity of air.

## Claims

1. Air cleaning apparatus for biological cleaning and filtration of air comprising a power source (23), an ionizer (5) with at least a pair of electrodes (6,7), the ionizer being connected to the power source (23), a fine filter (10;10';10'') disposed downstream of the ionizer (5) on an air flow path, and a coarse filter (1) disposed upstream of the ioniser (5) on the air flow path so that air passing through the ionizer (5) first passes through the coarse filter (1), which consists of at least three adjacent plates (2,4,3;2',4',3') through which the air flow passes, the at least three plates comprising an inner plate (4;4') of high-porous electret material between two outer plates (2,3;2',3') of gas permeable material, characterised in that the said electrodes (6,7) are electrically connected to respective terminals of opposite polarity of the power source (23), and the outer plates (2,3;2',3') are current conducting plates which are electrically connected to the respective terminals of opposite polarity of the power source (23) so that the coarse filter (1) functions as an electrostatic precipitator.

2. Apparatus according to claim 1, wherein the ionizer (5) comprises a needle-shaped discharge electrode (7) disposed along the air flow path, and a hollow, substantially cylindrical, non-discharging electrode (6) disposed coaxially relative to the discharge electrode (7) and wherein the outer plate (3) which is the nearest one to the ionizer (5) abuts on the end face of the cylindrical electrode (6) and overlaps it completely.

3. Apparatus according to claim 1 or 2, wherein the said gas-permeable current-conducting material is cellular metal having open porosity of at least 85% and pore diameter of 0.3 to 5 mm.

4. Apparatus according to any of claims 1 to 3, wherein the said electret material is polyurethane foam having open porosity of at least 85% and pore diameter of 0.3 to 5 mm.

5. Apparatus according to any of claims 1 to 3, wherein the fine filter (10) has a design similar to the design of the coarse filter (1).

6. Apparatus according to any of claims 1 to 5, wherein the fine filter (10';10'') has plates (13'-15'; 13''-15'') disposed in parallel with the direction of the air flow.

7. Apparatus according to claim 6, wherein the fine filter (10',10'') includes current-conducting plates (14',15'; 14'',15'') which are perforated, with holes (16;16'').

8. Apparatus according to claim 6 or 7, wherein the plates (13'',14'',15'') of the filter (10'') are shaped as cylinders disposed coaxially.

9. Apparatus according to any preceding claim, in which an additional ionizer (17) is disposed in series with the first-mentioned ionizer (5) and similar thereto, discharge electrodes (7,19) of the ionisers (5,17) being electrically connected to the terminals of opposite polarity of the power source (23).

10. Apparatus according to claim 9, wherein the ionizers have cylindrical non-discharging electrodes (18,21) of different internal diameters.

11. Apparatus according to claim 1, wherein the ionizer formed from a plurality of electrode pairs (24), all the non-discharging electrodes (25) being shaped as hexahedral prisms arranged compactly and oriented along the direction of the air flow, thus forming a honeycombed structure, the discharge electrodes (26) are needle-shaped and are disposed along the axial lines of the prisms, and the outer plate (3') of the coarse filter which is the nearest one to the ionizer abuts on the end faces of all the prisms (25) and overlaps them completely.

## Patentansprüche

1. Luftreinigungsvorrichtung zur biologischen Reinigung und Filtration von Luft mit einer Stromquelle (23), einem Ionisierer (5) mit mindestens einem Paar Elektroden (6, 7), wobei der Ionisierer mit der Stromquelle (23) verbunden ist, einem Feinfilter (10; 10'; 10''), das nach dem Ionisierer (5) an einem Luftstromweg angeordnet ist, und einem Grobfilter (1), das vor dem Ionisierer (5) an dem Luftstromweg so angeordnet ist, daß den Ionisierer (5) durchlaufende Luft zuerst das Grobfilter (1) durchläuft, das aus mindestens drei benachbarten Platten (2, 4, 3; 2', 4', 3') besteht, die der Luftstrom durchläuft, wobei die mindestens drei Platten eine Innenplatte (4; 4') aus porenreichem Elektretmaterial zwischen zwei Außenplatten (2, 3; 2', 3') aus gasdurchlässigem Material aufweisen, dadurch gekennzeichnet, daß die Elektroden (6, 7) mit jeweiligen Anschlüssen entgegengesetzter Polarität der Stromquelle (23) elektrisch verbunden sind und die Außenplatten (2, 3; 2', 3') stromleitende Platten sind, die mit den jeweiligen Anschlüssen entgegengesetzter Polarität der Stromquelle (23) elektrisch so verbunden sind, daß das Grobfilter (1) als elektrostatischer Abscheider wirkt.

2. Vorrichtung nach Anspruch 1, wobei der Ionisierer (5) aufweist: eine nadelförmige Entladungselektrode (7), die längs dem Luftstromweg angeordnet ist, und eine hohle, im wesentlichen zylindrische, nicht entladende Elektrode (6), die gleichachsig relativ zu der Entladungselektrode (7) angeordnet ist, und wobei die Außenplatte (3), die dem Ionisierer (5) am nächsten ist, an die Endfläche der zylindrischen Elektrode (6) anstößt und sie vollständig überdeckt.

3. Vorrichtung nach Anspruch 1 oder 2, wobei das gasdurchlässige stromleitende Material Zellenmetall mit einer offenen Porosität von mindestens 85 % und einem Porendurchmesser von 0,3 bis 5 mm ist.

4. Vorrichtung nach einem der Ansprüche 1 bis 3, wobei das Elektretmaterial Polyurethanschaum mit einer offenen Porosität von mindestens 85 % und einem Porendurchmesser von 0,3 bis 5 mm ist.

5. Vorrichtung nach einem der Ansprüche 1 bis 3, wobei das Feinfilter (10) eine Konstruktion hat, die der Konstruktion des Grobfilters (1) ähnelt.

6. Vorrichtung nach einem der Ansprüche 1 bis 5, wobei das Feinfilter (10'; 10'') Platten (13' bis 15'; 13'' bis 15'') hat, die parallel zu der Richtung des Luftstroms angeordnet sind.

7. Vorrichtung nach Anspruch 6, wobei das Feinfilter (10', 10'') stromleitende Platten (14', 15'; 14'', 15'') aufweist, die mit Löchern (16'; 16'') perforiert sind.

8. Vorrichtung nach Anspruch 6 oder 7, wobei die Platten (13'', 14'', 15'') des Filters (10'') als gleichachsig angeordnete Zylinder geformt sind.

9. Vorrichtung nach einem der vorstehenden Ansprüche, wobei ein zusätzlicher Ionisierer (17) in Reihe mit dem zuerst genannten Ionisierer (5) angeordnet und ihm ähnlich ist, wobei Entladungselektroden (7, 19) der Ionisierer (5, 17) mit den Anschlüssen entgegengesetzter Polarität der Stromquelle (23) elektrisch verbunden sind.

10. Vorrichtung nach Anspruch 9, wobei die Ionisierer zylindrische nicht entladende Elektroden (18, 21) mit unterschiedlichen Innendurchmessern haben.

11. Vorrichtung nach Anspruch 1, wobei der Ionisierer aus mehreren Elektrodenpaaren (24) gebildet ist, alle nicht entladenden Elektroden (25) als sechsflächige Prismen geformt sind, die kompakt angeordnet und längs der Richtung des Luftstroms orientiert sind, wodurch sie eine Wäbenstruktur bilden, die Entladungselektroden (26) nadelförmig und längs den Axiallinien der Prismen angeordnet sind und die Außenplatte (3') des Grobfilters, die dem Ionisierer am nächsten ist, an die Endflächen aller Prismen (25) anstößt und sie vollständig überdeckt.

## Revendications

1. Dispositif d'épuration d'air pour l'épuration et le filtrage biologiques de l'air comprenant une source de puissance (23), un ioniseur (5) avec au moins une paire d'électrodes (6, 7), l'ioniseur étant relié a la source de puissance (23), un filtre fin (10 : 10'; 10'') disposé en aval de l'ioniseur (5) sur un trajet d'écoulement d'air, et un filtre grossier (1) disposé en amont de l'ioniseur (5) sur le trajet d'écoulement d'air de manière que l'air traversant l'ioniseur (5) passe d'abord à travers le filtre grossier (1) qui consiste en au moins trois plaques adjacentes (2, 4, 3 ; 2', 4', 3') à travers lesquelles passe l'écoulement d'air, les au moins trois plaques comprenant une plaque intérieure (4, 4'), en un matériau électret a porosité élevée entre deux plaques extérieures (2, 3 ; 2', 3') d'un matériau perméable au gaz, caractérisé en ce que lesdites électrodes (6, 7) sont reliées électriquement à des bornes respectives de polarité opposée de la source de puissance (23) et en ce que les plaques extérieures (2, 3 ; 2', 3') sont des plaques conductrices de courant qui sont reliées électriquement aux bornes respectives de polarité opposée de la source de puissance (23) de telle manière que le filtre grossier (1) fonctionne comme organe de précipitation électrostatique.

2. Dispositif selon la revendication 1, dans lequel l'ioniseur (5) comprend une électrode de décharge en forme d'aiguille (7) disposée sur le trajet d'écoulement d'air et une électrode sans décharge, creuse sensiblement cylindrique (6), disposée coaxialement par rapport à l'électrode de décharge (7) et dans lequel la plaque extérieure (3) qui est la plus proche de l'ioniseur (5) est un butée sur la farce d'extrémité de l'électrode cylindrique (6) et la recouvre complètement.

3. Dispositif selon la revendication 1 ou 2, dans lequel ledit matériau conducteur de courant et perméable au gaz est du métal alvéolaire ayant une porosité ouverte d'au moins 85 % et un diamètre de pore de 0,3 à 5 mm.

4. Dispositif selon l'une quelconque des revendications 1 à 3, dans lequel ledit matériau électret est une mousse de polyuréthanne ayant une porosité ouverte d'au moins 85 % et un diamètre de pore de 0,3 à 5 mm.

5. Dispositif selon l'une quelconque des revendications 1 à 3, dans lequel le filtre fin (10) présente une structure similaire à celle du filtre grossier (1).

6. Dispositif selon l'une quelconque des revendications 1 à 5, dans lequel le filtre fin (10', 10'') présente des plaques (13' à 15': 13'' à 15'') disposées parallèlement à la direction de l'écoulement d'air.

7. Dispositif selon la revendication 10, dans lequel le filtre fin (10', 10'') comprend des plaques conductrices de courant (14', 15' ; 14'', 15'') qui sont perforées, avec des trous (16', 16'').

8. Dispositif selon la revendication 6 ou 7, dans lequel les plaques (13'', 14'', 15'') du filtre (10'') sont réalisées sous forme de cylindres disposés coaxialement.

9. Dispositif selon l'une quelconque des revendications précédentes, dans lequel un ioniseur supplémentaire (17) est disposé en série avec l'ioniseur (5) mentionné en premier et similaire à celui-ci, les électrodes de décharge (7, 19) des ioniseurs (5, 17) étant reliées électriquement aux bornes de polarité de polarité opposée de la source de puissance (23).

10. Dispositif selon la revendication 9, dans lequel les ioniseurs présentent des électrodes sans décharge cylindriques (18, 21) de diamètres internes différents.

11. Dispositif selon la revendication 1, dans lequel l'ioniseur est formé à partir d'une pluralité de paires d'électrodes (24), toutes les électrodes sans décharge (25) étant réalisées sous forme de prismes hexaédriques disposés de manière compacte et orientée selon la direction de l'écoulement d'air, en formant ainsi une structure en nid d'abeille, les électrodes de décharge (26) présentent une forme d'aiguille et sont disposées le long des lignes axiales des prismes, et la plaque extérieure (3') du filtre grossier qui est la plus proche de l'ioniseur vient en butée sur les faces d'extrémité de tous les prismes (25) et les recouvre complètement.
